# EUROPEAN PATENT APPLICATION

(11) **EP 3 527 666 A1**
(43) Date of publication of application: **21.08.2019**
(21) Application number: 19157119.9
(22) Date of filing: 14.02.2019
(51) Int. Cl.: C12P 21/00, A01N 25/00, A01N 63/00, C12P 7/64

(54) **PROCESS FOR OBTAINING BIOCIDE COMPOUNDS DERIVED FROM BACILLUS SP. CULTURES, FORMULATIONS THEREOF AND THEIR USE IN CONSERVATION OF OBJECTS OF CULTURAL HERITAGE**

(30) Priority: 14.02.2018 PT 2018110568
(71) Applicant: Universidade de Évora, 7000-803 Évora (PT)
(72) Inventor: CALDEIRA, ANA, 7000-651 EVORA (PT); CANDEIAS, ANTÓNIO, 7000-651 EVORA (PT); PEREIRA, ANTONIO, 7000-890 EVORA (PT); SILVA, MARA, 7005-313 EVORA (PT); ROSADO, TANIA, 7920-352 Vila Nova da Baronia (PT); MARTINS, SERGIO, 6060-124 IDANHA-A-NOVA (PT)
(74) Representative: Oliveira Lourenço, Nuno Miguel

(57) **Abstract**

The present invention relates a process for obtaining biocide compounds derived from *Bacillus* sp. cultures, compositions and formulations thereof, and their use in conservation of objects of cultural heritage.

The process of the present invention involves culturing and growing of three *Bacillus* sp. strains under nutrient stress conditions to obtain an enhanced production of lipopeptides (LPPs) having biocide activity.

Bioactive crude extracts of these *Bacillus* sp. cultures containing LPPs are disclosed herein and also bioactive compositions and formulations comprising said biocide compounds.

Said bioactive compounds, compositions and formulations have application in control the biodegradation, biodeterioration and conservation of objects of cultural heritage.

Therefore, the present invention is in the area of microbiology, cleaning of artworks, and conservation work of antique objects.

## Description

### TECHNICAL DOMAIN

The present invention relates a process for obtaining biocide compounds derived from *Bacillus* sp. cultures, compositions and formulations thereof, and their use in conservation of objects of cultural heritage.

The process of the present invention involves culturing and growing of three *Bacillus* sp. strains under nutrient stress conditions to obtain an enhanced production of lipopeptides (LPPs) having biocide activity.

Bioactive crude extracts of these *Bacillus* sp. cultures containing LPPs are disclosed herein and also bioactive compositions and formulations comprising said biocide compounds.

Said compounds, compositions and formulations have application in the control of biodegradation, biodeterioration and conservation of objects of cultural heritage.

Therefore, the present invention is in the area of microbiology, cleaning of artworks, and conservation work of antique objects.

### STATE OF THE ART

To control the biodegradation/biodeterioration process in cultural heritage context different approaches can be used, as such as: indirect control by altering environmental conditions, mechanical removal of biodeteriogens, physical eradication methods and chemical/biocidal treatment.

The mechanical methods to clean the surfaces includes the use of scalpels, spatulas, scrapers and vacuum cleaners. This approach does not add new material on the substrates, there is a danger of cells or microbial spores remaining on the material, which are able to reactivate in favourable conditions and induce alteration processes.
The physical methods are also being applied with this purpose and are based on the application of ultraviolet light, gamma rays, high-frequency current and low-frequency electrical current. However, low temperatures, pressure reduction and ultrasound can also be used.

The most efficient method is irradiation with UV between 200-300 nm, however it is necessary to take into account that UV light has a poor penetration power and can lead photodegradation of pigments and organic materials.

The use of UV-C irradiation is an alternative to chemical products because this process does not generate pollution phenomena and the physical support remains unaltered. UV-C irradiation is harmful to living organisms due to its short wavelength, which confers highly energetic photons and germicidal properties upon these organisms, compromising the viability and metabolic activity of the microorganisms.

Gamma-irradiation can have several advantages for the conservation of objects of cultural heritage. It is highly penetrating and therefore very efficient in killing microbial communities colonising these objects. Furthermore, this technique is of use to conservators as it is not producing hazardous traces for paintings, it does not cause the formation of secondary radioactivity nor the formation of toxic residues and it is cost attractive. The required dose of gamma irradiation depends on the contamination level, the microbial diversity and its capacity for irradiation resistance. Nevertheless, gamma irradiation is not suitable for large paintings and it does not have a long-lasting effect. Beyond this limitation, a major problem in using gamma irradiation to eliminate colonising microorganisms is the possible deterioration of the object to preserve. The colour stability might be affected as chemical and physical properties of pigments may be changed due to gamma irradiation.

Laser cleaning, in comparison with conventional mechanical and chemical cleaning is a precise and versatile non-contact method. It has lower environmental and health-related side effects and prevents damage to underlying substrates by through self-controlling mechanisms. The cleaning of artworks by laser irradiation is a contemporary technique with many practical advantages (precision, rapidity, localised action, etc.). The main problem in the case of the biological degradation of artworks is a complete elimination of biodeteriogens and the treatment efficiency (removal of all active organisms).

The chemical treatments frequently applied have high efficiency in the microbial elimination, however, the main problem is the toxicity of the compounds used. The commercial biocides available are mainly alcohols, aldehydes, organic acids, carbon acid esters, phenols and their derivatives, halogenated compounds, metals and metal-organic substances, among others. Compounds like quaternary ammonium salts, metals and metal organic substances and heterocyclic organic products, have been widely applied for the control of microbial growth on artworks.

Among the products currently used, quaternary ammonium salts are a group of substances widely applied in artworks treatment due to its broad-spectrum action and low toxicity. The antimicrobial effect of quaternary ammonium compounds is probably based on the inactivation of proteins and enzymes and the detrimental impact on the microbial cell membrane. Their effectiveness is dependent on their chemical structure, such as the presence of an aromatic ring structure and the respective length of the four radicals. These compounds affect a broad microbial spectrum ranging from bacteria, fungi to algae and lichens, however possess high toxicity levels.

Thus, in general, one biocide must to efficiently kill all microorganisms, have no/low toxicity for the operator and a low risk for the environment, does not interfere with materials, and offer protection against microorganisms for a long period of time.

In fact, the efficiency of a chemical treatment depends on the concentration, microbial diversity and persistence of its action through time. There are different ways of application as such as spraying, brushing, applying poultices, injection or fumigation.

Biocidal treatments have to be undertaken during dry weather. In windy conditions, excessive unwanted removal of biocidal spray may pose health and environmental risks. These compounds are commonly applied in repairing, cleaning and maintenance of artworks. Their application aims to prevent and/or control microbial growth. In this way, biocides can be applied before conservation-intervention process to eliminate microorganisms already present, and, after the intervention as preventive effect to slow down the re-colonisation of restored surfaces.

Although the inhibition capacity, these biocides possess high toxicity which can represent an environmental risk. In this way, besides the ability to control biological growth against biological agents, the requirements for a good biocide are: high effectiveness against biodeteriogens, absence of interference with the constituent materials, low toxicity to human health and low risk of environmental pollution.

In this sense, a greater effort has been undertaken to develop safer, environmental friendly, less costly and highly effective management methods that pose less risk to these monuments and, consequently, to humans (usually visitors) and the environment.

Due to the limitations related with the use of chemical compounds, natural products represent a huge potential source of compounds with antimicrobial properties, which can be an useful and advantageous alternative for the chemical products.

Therefore, the present invention proposes biocide formulations based on natural products, such as Bacillus sp. strains to solve the problems stated above and produce a safe, efficient, environmental friendly and low cost products to be applied to conservation of artworks without negative effects on the environment or human beings.

### SUMMARY OF THE INVENTION

The present invention relates a process for obtaining biocide compounds derived from *Bacillus* sp. cultures, compositions and formulations thereof, and their use in conservation of objects of cultural heritage.

The process of the present invention involves culturing and growing of three *Bacillus* sp. strains under nutrient stress conditions to obtain an enhanced production of lipopeptides (LPPs) having biocide activity according to claim 1.

In a second aspect, the present invention relates to a bioactive crude extract solution prepared according to the process of the invention as described in claim 7.

In a third aspect, the present invention relates to a bioactive composition prepared according to the process of the invention as described in claim 8.

In a fourth aspect, the present invention relates to a bioactive formulation prepared according to the process of the invention as described in claim 9.

In another aspect, the present invention related to the uses of bioactive crude extracts, bioactive compositions and bioactive formulations for controlling of biodegradation, biodeterioration and conservation of objects of cultural heritage according to claim 10.

The inventive formulations possess equal or higher inhibition capacity than chemical comparative formulations even in some cases the inventive formulations presenting total inhibition of fungal growth.

Besides the very good inhibition results produced by the inventive formulations, results also show that these also present an ecological, safe and green solution face to the commercial compounds available on the market.

Therefore, the process of the present invention allows an important contribution to the development of new mitigation approaches for cultural heritage safeguard and highlights the advantages of obtaining novel biocompounds, compositions and formulations to chalenge the biodegradation/bioderioration problem.

The efficiency of the novel and ecofriendly antifungal compositions and formulations of the invention constitute an alternative to usual toxic compounds and can be integrated on mitigation strategies to promote the safeguard of for cultural heritage artefacts.

### DESCRIPTION OF THE FIGURES

**Fig.1****.** Time course profile of Bacillus sp. CCLBH 1053 culture in NB medium. All data was determined in triplicate. I-Lag phase; II-Log phase; III-Stationary phase.
   The time course profile of Bacillus sp. CCLBH 1053 culture in NB medium show a lag phase of about 2 hours, a period of exponential growth of approximately 8-10 hours and a stationary phase, after 11 hours of culture.
**Fig.2****.** Antifungal activity of bioactive formulation BEVOTECH-16 comprising LPPs produced by *Bacillus* sp. CCLBH 1053 against *P. glandicola* CCLBH-MP101, *A. brasiliensis* CCLBH-MP201 and *F*. *buharicum* CCLBH-MP301, where it can be observed that said bioactive formulation has a particular potent effect against *P. glandicola* CCLBH-MP101.
**Fig.3****.** Shows the antifungal activity of a bioactive composition of the invention produced by *Bacillus* sp. CCLBH 1053 and commercial antifungal drugs against the biodeteriogenic fungi, isolated from deteriorated mural paintings.
**Fig.4****.** Shows marble slabs, SEM micrographs of the MCP and MP micro-fragments **(a), (b)** and EDX analysis **(d), (c). (a), (c):** MCP slabs inoculated with *P. glandicola* CCLBH-MP101.; **(b), (d):** MP slabs treated with inventive compositions produced by *Bacillus* sp. CCLBH 1053.

### DESCRIPTION

The present invention relates to biocide compounds, compositions and formulations derived from Bacillus sp. cultures and to a process for obtaining them. Said compounds, compositions and formulations have application in the control of biodegradation, biodeterioration and conservation of objects of cultural heritage.

Natural substances with antimicrobial action have been identified from a very wide range of sources, including plants, microorganisms and animals. In this way, several strains of *Bacillus subtilis* and *Bacillus amyloliquefaciens* have been referred to produce lipopeptides (LPPs).

In response to nutritional stress, a variety of processes are activated by *Bacillus* strains, including sporulation, synthesis of extracellular degradative enzymes and lipopeptides production (LPP).

LPP may be regarded as green solutions, whose added value is to be eco-friendly and without negative effects on the environment or human beings, and for the artworks.

For this purpose, 3 strains of *Bacillus sp.,* namely *Bacillus amyloliquefaciens* CCLBH 1051, CCLBH 1052 and CCLBH 1053 were selected and grown for their ability to produce LPPs in adequate culture media in order to induce nutrient stress conditions and to produce said active compounds of interest.

Said active compounds are recovered and used to make a crude bioactive solution (identified herein as Crude-C solution) in a solvent system solution. The bioactive compounds are then separated by chromatographic techniques. The resulting bioactive compositions (identified herein as Ly-Tech compositions) can be dried, lyophilized and distributed in vials for storage and later used to produce the biocide formulations of the invention (herein identified as BEvoTech formulations).

In short, the process of the invention comprises the following steps:
a) Culturing and growing of *Bacillus amyloliquefaciens* strains CCLBH 1051, CCLBH 1052 and CCLBH 1053 in an adequate culture medium to induce nutritional stress, during 72h for producing LPP compounds;
b) Removal of Bacillus cells from the cultured medium;
c) Lyophilisation of the resulting cell-free supernatant of step (b);
d) Resuspension of the lyophilized product of (c) in a chloroform/methanol/water solvent-system solution to obtain a crude bioactive solution (Crude-C solution);
e) Chromatographic separation of the relevant bioactive compounds, namely the LPP compounds of the Crude C solution of step (d)

The resulting bioactive solutions of step (e) can be further dried and subsequently lyophilized to obtain **bioactive compositions Ly-Tech-1051, Ly-Tech-1052 and Ly-Tech-1053,** respectively obtained from cultures of Bacillus sp. CCLBH 1051, CCLBH 1052 and CCLBH 1053.

A further step can be added to the process above described to obtain **BEVOTECH bioactive formulations** by addition of **Ly-Tech compositions** to polypropylene glycol 2000 and water, in a proportion of 400mg Ly-Tech composition to 2 ml polypropylene glycol 2000 (2%, v/v) and 998 water, to form BEVOTECH-11, BEVOTECH-14 and BEVOTECH-16, respectively comprising **Ly-Tech-1051, Ly-Tech-1052 and Ly-Tech-1053** as described above, where the polypropylene glycol 2000 (PPG) is identified with CAS Number: 25322-69-4.

The above mentioned steps can be performed as the following:

### I. Microorganism and culture media

### 1. Growth conditions

The *Bacillus sp.* (*Bacillus amyloliquefaciens* CCLBH 1051, CCLBH 1052 and CCLBH 1053) cells grows in batch culture at 30°C using a bioreactor with a sterilized adequate culture medium to induce nutritional stress, such as CM_B.

CM_B culture media contains per litre: (NH4)2SO4, 1.5 g; KH2PO4, 1.7 g; Na2HPO4·2H2O, 1.7 g; MgSO4·7H2O, 0.2 g; yeast extract, 0.1 g; glucose 2 g and 2mL of micronutrients solution (MS) sterilised separately. MS containing per litre: EDTA 25,0g; ZnSO4.7H2O 4,5g; MnCl2.4H2O 13,0g; CoCl2.6H2O 0,3g; CuSO4.5H2O 0,3g; NaMoO4.2H2O 0,4g; CaCl2.2H2O 4,5g; FeSO4.7H2O 3,0g; H3BO3 1,0g; and KI 0,1g.

The inoculum culture is incubated for 24 h at 30°C in an orbital shaker at 200rpm. Control of foam can be performed by the addition of 2mL/L of medium of a sterilized aqueous solution of polypropylene glycol 2000 (2%, v/v).

The bioreactor is inoculated with 100mL stationary-phase culture. The culture pH can be measured by a combined electrode and controlled to a value of 7±0.2 by addition of basic solutions, such as NaOH 2M, and acid solutions, such as H₂SO₄ 1M. Preferably, pH control is done in an automatic way through peristaltic pumps fitted in the control unity.

Temperature is measured and settled around 30°C preferably by using a platinum thermocouple (Pt-100) temperature sensor and was controlled at +/- 30°C by a water thermos-circulator, in a double jacket present in the vessel. The dissolved oxygen level was measured by a polarographic electrode. The stirring speed was set to +/- 700rpm and the aeration rate was +/- 2 vvm.

### 2. Culture monitoring

To monitor the *Bacillus* sp. (CCLBH 1051, CCLBH 1052 and CCLBH 1053) cells growth, the absorbance is periodically monitored at 600 nm.

The kinetics of the bacterial populations growth was modelled by the Gompertz model expressed by: log N/N0 = A. exp (-exp (-b-cx)), where the N is the decimal logarithm of microbial counts (log (CFU/mL)) at time t; N0 is the asymptotic log count as time decreases indefinitely (log (CFU/mL)); A is the number of log cycles of growth (log(CFU/mL)); b is the relative growth rate at time (h-1) and c is the time required to reach the maximum growth rate (h), as described by Chowdhury et *al.* (Chowdhury, B. R.; Chakraborty, R.; Chaudhuri, U. R., Validity of modified Gompertz and Logistic models in predicting cell growth of Pediococcus acidilactici H during the production of bacteriocin pediocin AcH. Journal of Food Engineering 2007, 80 (4), 1171-1175).

The specific growth rate, µ were determined from experimental data in exponential phase, using the relation µ(h-1) = (c. A)/e, where µ is the specific growth rate of the microorganism (log (CFU/mL)/h) and e is the neper number. The time of generation (g) was calculated by g (h) = Ln (2)/µ, according to Zwietering et al. (Zwietering, M. Jongenburger, I.; Rombouts, F.; Van't Riet, K., Modeling of the bacterial growth curve. Applied and Environmental Microbiology 1990, 56 (6), 1875-1881).

The specific growth rate and the generation time for the e strains was determined from the exponential growth period, displaying a specific growth rate (µ) of 0.603 ± 0.056 h-1 and a generation time (g) of 1.149 ± 0.095 h, respectively.

The time course profile of Bacillus sp. strains in NB medium show a lag phase of about 2 hours, a period of exponential growth of approximately 8-10 hours and a stationary phase, after 11 hours of culture.

### II. Active compounds recovery

After 72h of culture cells were removed and cell-free supernatant cultures were lyophilized. The resulting powder is used to prepare 40mg/mL solution (identified as Crude-C solution) in a solvent system such as chloroform/methanol/water (65/35/5) solvent system. The bioactive compounds are recovered from 50 mL of Crude-C solution (or proportional amounts) after chromatographic separation. Adequate chromatographic conditions are for example, a stationary phase of flash silica gel 60 (0.04-0.06mm) in a glass column with 5cmx30cm. The elution is performed at +/- 20ml/min, during +/- 10 minutes.

### III. Bioactive compositions (Ly-Tech)

The resulting eluate or Crude-C solutions can be dried at 40°C preferably in a rotary evaporator and subsequently lyophilized, giving three compositions identified as (1) Ly-Tech-1051, (2) Ly-Tech-1052 and (3) Ly-Tech-1053, respectively to cultures of *Bacillus sp.* CCLBH 1051, CCLBH 1052 and CCLBH 1053. Ly-Tech compositions are used to produce BEvoTech formulations.

### IV. Bioactive formulations (BEvoTech)

Three formulations comprising the above mentioned Ly-Tech products were obtained, namely:
- BEVOTECH-11, comprising 400mg of Ly-Tech-1051 composition + 2 ml polypropylene glycol 2000 (2%, v/v) + 998 water;
- BEVOTECH-14, comprising 400 mg of Ly-Tech-1052 composition + 2 ml polypropylene glycol 2000 (2%, v/v) + 998 water; and
- BEVOTECH-16, comprising 400mg of Ly-Tech-1053 composition + 2 ml polypropylene glycol 2000 (2%, v/v) + 998 water.

### V. Antifungal inhibitory capacity assessment

The biocide properties of the compositions and formulations of the present invention, as described above, were tested against fungi strains isolated from several objects of cultural heritage, such as mural paintings, marble slabs.

Fungal inhibition was tested in the presence of different commercial (comparative compounds) and compositions and/or formulations of the invention, obtained as described above.

For comparative purposes, the activity of Micostatin® (Nystatin 4000 UI/mL) and Preventol PN® 0.1% [sodium 2, 3, 4, 5, 6-pentachlorophenplate] was also evaluated.
All comparative compounds, and inventive compositions and formulations were tested at different concentrations against several fungal isolates from heritage context.

Three biodeteriogenic fungi strains isolated from biodegraded mural paintings *Penicillium glandicola* CCLBH-MP101, *Aspergillus brasiliensis* CCLBH-MP201 and *Fusarium buharicum* CCLBH-MP301 were used to study the antifungal potential of the compositions and/or formulations of the invention.

Cultures of each microorganism were prepared in Malt Extract Agar (MEA) slants and incubated at 28°C for 7 days. Fungal spore suspensions were prepared by adding a loopful of hyphae and spores in 15 mL of NaCl 0.85% solution. The suspension was filtered by sterilised triple gauze and incorporated (10⁵ CFU/mL) in MEA at 45°C. Sterile filter paper discs (Macherey-Nagel 827 ATD) were placed on agar and impregnated with 20 µL of biocide. The Petri dishes were incubated at 28°C for 4-5 days.

Antimicrobial activity was evaluated accordingly to the inhibition halo developed around the disc. The assays were performed in triplicate, and the results presented as average ± standard deviation (SD).

Data was evaluated statistically using the SPSS® 24.0 software for Windows Copyright®, Microsoft Corporation, by descriptive parameters and by One-way ANOVA in order to determine statistically significant differences at the 95% confidence level (p<0.05). The population variances homogeneity was confirmed by Levene test and multiple average comparisons were evaluated by Tukey test, being considered significant values those whose probability of occurrence is greater than 95% (p<0.05).

Results show that the compositions and/or formulations of the invention possess equal or higher inhibition capacity than conventional chemical products (comparative compounds) and even in some cases are able of producing total inhibition of the fungal culture tested.

Marble samples were used to evaluate real life efficiency and influence of the compositions and/or formulations of the invention on the activity of biodeteriogenic microorganisms present in said samples by performing analyses under in situ controlled conditions.
After sterilization, marble slabs were inoculated with mixtures comprising *Penicillium glandicola* CCLBH-MP101 (MP), 105 CFU/mL spore suspension, respectively, 500 µL of malt extract and 500 µL of one of each of the *Bacillus sp.* liquid culture supernatant, produced as described above, comprising the bioactive compounds produced. The method used is illustrated below, in **scheme 1.**

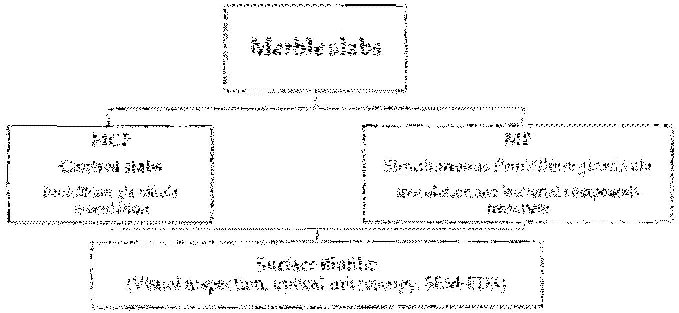

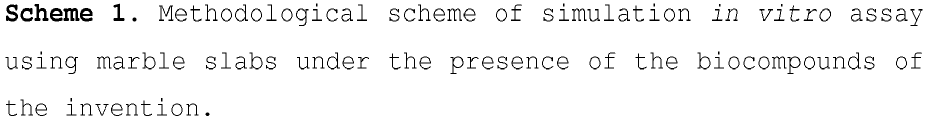

The slabs were incubated at 27°C, monitored periodically and documented using a digital camera. After 1 month of incubation all the slabs were air-dried, coated with gold and examined by Scanning Electron Microscopy (SEM) using a Hitachi S-3700N (Tokyo, Japan) variable pressure scanning electron microscope coupled to a Bruker XFlash 5010 energy dispersive X-ray (EDX) spectrometer (Berlin, Germany) to allow visualization of the surface "biofilm" and elemental composition (point analysis and two-dimensional mapping). The accelerating voltage was 18-20 kV.

In order to develop and study the real efficiency and influence of the compounds in the biodeteriogenic fungi development, an assay using marble slabs were envisaged. Slabs were inoculated with *P*. *glandicola* CCLBH-MP101 and treated with the compositions and/or the formulatiions of the invention under *in situ* controlled conditions. Table 2 shows the macroscopic features corresponding to the marble slab assays, in the presence of *P. glandicola* CCLBH-MP101, a common fungi genera found in biodegraded artworks.

After 6 days of marble slab treatment, the positive control (MCP) begins to exhibit stains in the slab surface, marking the start of fungal proliferation. At the end of the assay and after 30 days of incubation, the MCP slabs show evident contamination sports. On the contrary, the MP slabs, treated with the biocompounds of the invention, indicate visually a few signals of microbiological contamination in it surface and a marked difference with the slabs without antifungal treatment (MCP) .

The MCP slabs exhibited a clear presence of microbial cells thriving in the stone. It was possible to observe spores and hyphae penetrating into the microstructure of the marble, promoting the proliferation of these microorganisms. Also, the EDX elemental analysis showed the presence of chemical elements such as carbon and oxygen, confirming the presence of organic material in the slabs.

Relatively to the treated MP slabs with the biocompounds of the invention, a few evidences of fungal growth were observed by SEM.

The lack of fungal proliferation was confirmed by the EDX analyses that only showed the presence of marble nature composition elements, sodium (Na), chlorine (Cl) and calcium (Ca) .

These simulation assays evidenced the great potential of the bacterial bioactive compounds to inhibit fungal proliferation in marble materials.

### VI. Antagonistic effect of BEVOTECH-11, BEVOTECH-14 and BEVOTECH-16 against Fungi

Fungal inhibition was tested in the presence of different commercial (comparative formulations) and bioactive formulations of the invention BEVOTECH-11, BEVOTECH-14 and BEVOTECH-16 obtained by biotechnological methodologies, using different strains of *Bacillus* sp., referred to produce bioactive compounds which has a great potential to suppress biodeteriogenic fungi growth.

A positive control Micostatin® (Nystatin 4000 UI/mL) and commercial chemical compounds Preventol PN® 0.1% [sodium 2, 3, 4, 5, 6-pentachlorophenplate] was also evaluated. The comparative and inventive lipopeptide compounds were tested at different concentrations against several fungal isolates from heritage context.

Cultures of each microorganism were prepared in Malt Extract Agar (MEA) slants and incubated at 28°C for 7 days. Fungal spore suspensions were prepared by adding a loopful of hyphae and spores in 15 mL of NaCl 0.85% solution. The suspension was filtered by sterilised triple gauze and incorporated (10⁵ CFU/mL) in MEA at 45°C. Sterile filter paper discs (Macherey-Nagel 827 ATD) were placed on agar and impregnated with 20 µL of biocide. The Petri dishes were incubated at 28°C for 4-5 days. Antimicrobial activity was evaluated accordingly to the inhibition halo developed around the disc. The assays were performed in triplicate, and the results presented as average ± standard deviation (SD).

Data were evaluated statistically using the SPSS® 24.0 software for Windows Copyright®, Microsoft Corporation, by descriptive parameters and by One-way ANOVA in order to determine statistically significant differences at the 95% confidence level (p<0.05). The population variances homogeneity was confirmed by Levene test and multiple average comparisons were evaluated by Tukey test, being considered significant values those whose probability of occurrence is greater than 95% (p<0.05).

Table 1 presents the results corresponding to commercial (comparative) and natural (inventive) formulations against filamentous fungi isolated from heritage artefacts.

**Table 1. Antifungal activity of commercial and natural compounds against filamentous fungi isolated from Heritage context.**

| **Fungal isolates** | | **Inhibition halo (mm)** | | | | |
|---|---|---|---|---|---|---|
| | | **Nystatin** | **Prev. 0.1%** | **BEV-11** | **BEV-14** | **BEV-16** |
| CC_1 | *Cladosporium* sp. 1 | 12.7 ± 0.5 ^{a} | N.I. ^{b} | 18.4 ± 1.3 ^{c} | 15.2 ± 0.4 ^{d} | 14.3 ± 0.7 ^{d} |
| CC_2 | Mycellium 1 | 17.7 ± 2.8 ^{a} | 16.9 ± 2.1 ^{a} | 22.6 ± 1.0 ^{b} | 20.2 ± 1.6 ^{b,c} | 19.2 ± 2.1 ^{a,c} |
| CC_3 | *Aspergillus* sp.1 | 17.1 ± 1.5 ^{a} | 16.0 ± 1.0 ^{b} | 15.8 ± 0.7 ^{a} | 15.1 ± 0.9 ^{a,c} | 13.6 ± 0.7 ^{d} |
| CC_4 | *Penicillium* sp.1 | 16.1 ± 1.7 ^{a} | T.I. ^{b} | 30.1 ± 1.9 ^{c} | 28.0 ± 3.7 ^{c} | 22.9 ± 3.1 ^{d} |
| CC_5 | Mycellium 2 | 23.4 ± 2.8 ^{a} | 21.3 ± 2.8 ^{a} | 24.8 ± 2.4 ^{a} | 26.6 ± 3.4 ^{a} | 24.7 ± 3.9 ^{a} |
| CC_6 | *Penicillium* sp.2 | 18.3 ± 2.5 ^{a} | 27.1 ± 2.1 ^{b} | 23.9 ± 3.6 ^{c} | 27.8 ± 2.5 ^{b,d} | 18.8 ± 1.5 ^{a} |
| CC_7 | *Cladosporium* sp. 2 | 16.1 ± 4.2 ^{a} | 15.0 ± 0.9 ^{a} | 27.0 ± 1.5 ^{b} | 27.8 ± 1.2 ^{b} | 21.8 ± 1.4 ^{c} |
| CC_8 | Mycellium 3 | 19.9 ± 2.5 ^{a} | 27.7 ± 5.2 ^{b} | 35.4 ± 4.2 ^{c} | 41.4 ± 8.8 ^{d} | 31.4 ± 5.5^{b,c,e} |
| CC_9 | *Penicillium* sp.3 | 17.3 ± 3.9 ^{a} | 14.0 ± 0.9 ^{b} | 33.2 ± 1.7 ^{c} | 29.7 ± 1.7 ^{d} | 22.2 ± 1.4 ^{e} |
| CC_10 | Mycellium 4 | 16.1 ± 1.8 ^{a} | 28.1 ± 8.4 ^{b} | 35 ± 3.6 ^{c} | 30.3 ± 1.9^{b,c} | 22.4 ± 1.9 ^{d} |
| CC_11 | *Acremonium* sp. 1 | 14.2 ± 0.9 ^{a} | 23.2 ± 3.3 ^{b} | 38.4 ± 1.9 ^{c} | T.I. ^{d} | 41.9 ± 2.4 ^{e} |
| CC_12 | *Penicillium* sp.4 | 21.4 ± 4.0 ^{a} | T.I. ^{b} | 34.8 ± 3.3 ^{c} | 32.3 ± 3.3 ^{c} | 22.0 ± 1.7 ^{a} |
| CC_13 | *Acremonium* sp.2 | 15.1 ± 1.3 ^{a} | 20.7 ± 1.2 ^{b} | T.I. ^{c} | T.I. ^{c} | 40.2 ± 0.8 ^{d} |
| CC_14 | Mycellium 5 | 14 ± 2.1 ^{a} | 19.4 ± 1.2 ^{b} | 38.8 ± 1.1 ^{c} | 35 ± 2.7 ^{d} | 29.7 ± 2.2 ^{e} |
| CC_15 | *Mycellium 6* | 12 ± 0.0 ^{a} | 15.8 ± 0.7 ^{b} | 27.3 ± 1.7 ^{c} | 24.1 ± 1.2 ^{d} | 21.7 ± 1.0 ^{e} |
| CC_16 | *Penicillium sp.5* | 22.1 ± 1.9 ^{a} | 15.7 ± 1.5 ^{b} | 32.2 ± 2.8 ^{c} | 34.7 ± 2.6 ^{c} | 22.6 ± 2.2 ^{a} |
| CC_17 | *Penicillium* sp.6 | 25.6 ± 2.4 ^{a} | 13.0 ± 0.0 ^{b} | 22.9 ± 1.4 ^{c} | 22.3 ± 1.2 ^{c} | 17.0 ± 0.7 ^{d} |

| | | | | | | |
|---|---|---|---|---|---|---|
| T.I. - total inhibition; N.I. - no inhibition * Different letters following the values indicate significant differences (p<0.05) for each isolate in the presence of the several biocides tested. Values of each determination represents means ± SD (n=9). | | | | | | |

The inventive formulations possess equal or higher inhibition capacity than chemical comparative formulations even in some cases the inventive formulations presenting total inhibition of fungal growth.

Besides the very good inhibition results produced by the inventive formulations, results also show that these also present an ecological, safe and green solution face to the commercial compounds available on the market.

Therefore, the process of the present invention allows an important contribution to the development of new mitigation approaches for cultural heritage safeguard and highlights the advantages of obtaining novel biocompounds, compositions and formulations to challenge the biodegradation/bioderioration problem.

The efficiency of the novel and eco-friendly antifungal compositions and formulations of the invention constitute an alternative to usual toxic compounds and can be integrated on mitigation strategies to promote the safeguard of for cultural heritage artefacts.

### EXAMPLES

### Example 1. Bacillus sp. strains culture and growth

The selected Bacillus sp. strains *Bacillus amyloliquefaciens* CCLBH 1051, CCLBH 1052 and CCLBH 1053 were cultivated and grown in batch culture at 30°C using a bioreactor with a sterilized culture medium (CM_B).

CM_B containing per litre: (NH4)2SO4, 1.5 g; KH2PO4, 1.7 g; Na2HPO4·2H2O, 1.7 g; MgSO4·7H2O, 0.2 g; yeast extract, 0.1 g; glucose 2 g and 2mL of micronutrients solution (MS) sterilised separately. MS containing per liter: EDTA 25,0g; ZnSO4.7H2O 4,5g; MnCl2.4H2O 13,0g; CoCl2.6H2O 0,3g; CuSO4.5H2O 0,3g; NaMoO4.2H2O 0,4g; CaCl2.2H2O 4,5g; FeSO4.7H2O 3,0g; H3BO3 1,0g; and KI 0,1g.

The inoculum culture was incubated for 24 h at 30°C in an orbital shaker at 200rpm. Control of foam was performed by the addition of 2mL/L of medium of a sterilized aqueous solution of polypropylene glycol 2000 (2%, v/v).

The bioreactor was inoculated with 100mL stationary-phase culture. The culture pH is measured by a combined electrode and controlled to 7±0.2 by the automatic addition of base (NaOH 2M) and acid solutions (H₂SO₄ 1M) through peristaltic pumps fitted in the control unity. Temperature is measured by using a platinum thermocouple (Pt-100) temperature sensor and was controlled at 30°C by a water thermos-circulator, in a double jacket present in the vessel.

The dissolved oxygen level was measured by a polarographic electrode. The stirring speed was set to 700rpm and the aeration rate was 2 vvm.

### Example 2. Culture monitoring

To monitor the *Bacillus* sp. (CCLBH 1051, CCLBH 1052 and CCLBH 1053) the absorbance of cells growth in the conditions according to the described in Example 1, was periodically monitored at 600 nm and their time course profile was drawn.

The kinetics of the bacterial populations growth was modelled by the Gompertz model expressed by: log N/N0 = A. exp (-exp (-b-cx)), where the N is the decimal logarithm of microbial counts (log (CFU/mL)) at time t; N0 is the asymptotic log count as time decreases indefinitely (log (CFU/mL)); A is the number of log cycles of growth (log(CFU/mL)); b is the relative growth rate at time (h-1) and c is the time required to reach the maximum growth rate (h).

The specific growth rate, µ was determined from experimental data in exponential phase, using the relation µ (h-1) = (c. A)/e, where µ is the specific growth rate of the microorganism (log (CFU/mL)/h) and e is the neper number. The time of generation (g) was calculated by g (h) = Ln (2)/µ, wherein it presents: a lag phase of about 2 hours, a period of exponential growth of approximately 8-10 hours and a stationary phase, after 11 hours of culture.

The specific growth rate and the generation time for the e strains was determined from the exponential growth period, displaying a specific growth rate (µ) of 0.603 ± 0.056 h-1 and a generation time (g) of 1.149 ± 0.095 h, respectively.

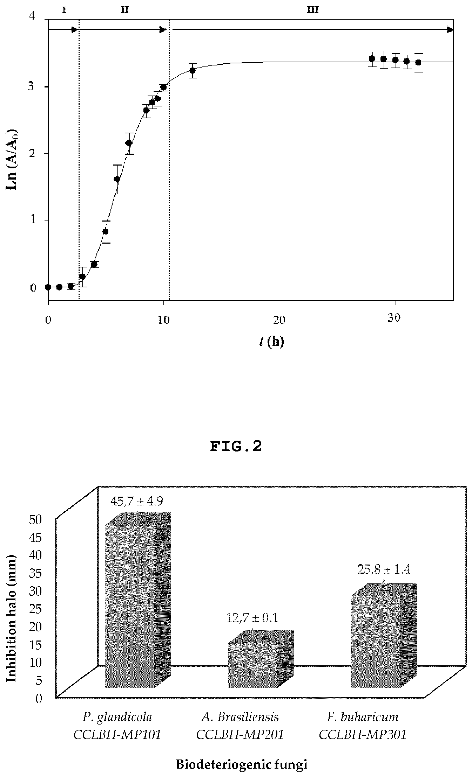

Fig. 1 shows the time course profile of *Bacillus sp.* CCLBH 1053 in NB medium showing a lag phase of about 2 hours, a period of exponential growth of approximately 8-10 hours and a stationary phase, after 11 hours of culture.

### Example 3. Bioactive compounds recovery

After 72h of culture bacterial cells were removed and cell-free supernatant cultures were lyophilized. The resulting powder is used to prepare a 40mg/mL solution (Crude-C) in a chloroform/methanol/water (65/35/5) solvent system.

The bioactive compounds were recovered from 50 mL of Crude-C solution after chromatographic separation, using as stationary phase of flash silica gel 60 (0.04-0.06mm) in a glass column with 5cmx30cm. The elution was performed at 20ml/min, during 10 minutes.

### Example 4. Ly-Tech Bioactive compositions

The resulting eluate (Crude-C solution) was dried at 40°C in a rotary evaporator and subsequently lyophilized, giving the compositions Ly-Tech-1051, Ly-Tech-1052 and Ly-Tech-1053, respectively to cultures of Bacillus sp. CCLBH 1051, CCLBH 1052 and CCLBH 1053. Ly-Tech products were used to produce BEvoTech formulations.

### Example 5. BEVOTECH Formulations of the invention

Formulations were produced by using Ly-Tech products as described in Example 3 resulting in the following formulations:
- BEVOTECH-11: 400mg Ly-Tech-1051 + 2 ml polypropylene glycol 2000 (2%, v/v) + 998 water;
- BEVOTECH-14: 400 mg Ly-Tech-1052 + 2 ml polypropylene glycol 2000 (2%, v/v) + 998 water; and
- BEVOTECH-16: 400mg Ly-Tech-1053 + 2 ml polypropylene glycol 2000 (2%, v/v) + 998 water.

### Example 6. Antifungal inhibitory capacity of Ly-Tech-1053 against fungus from mural paintings

Ly-Tech-1053 produced by Bacillus sp. CCLBH 1053 cultures in the stationary-phase of bacteria growth was prepared as described in Example 4 and tested against *Penicillium glandicola* CCLBH-MP101, *Aspergillus brasiliensis* CCLBH-MP201 and *Fusarium buharicum* CCLBH-MP301, biodeteriogenic fungal strains isolated from biodegraded mural paintings for assessment of its antifungal activity.

Fungal spore suspensions were prepared by adding loopful of hyphae and spores from a Malt Extract Agar (MEA) slant incubated at 25°C for 7 days, in 5 mL of NaCl 0.85% solution. The suspensions were filtered by sterilized cotton or triple gauze. A 106 CFU/mL spore suspensions were obtained through dilutions and fungal suspensions were incorporated in MEA at 45°C in Petri dishes. Filter paper discs (Macherey-Nagel 827 ATD) impregnated with 10 µL of Bacillus culture broth, after cells removal were placed on the agar and the Petri dishes were incubated at 25°C for 24-48 h.

The antifungal activity was evaluated by the formation and the size of inhibition halos around the discs containing the biocide compound.

For comparative purposes, commercial antifungal compounds Nystatin (1 mg/mL) were also used as positive control.

Inhibition halos were formed in all tested fungus demonstrating the antifungal potential of the produced lipopeptides present in Ly-Tech-1053 exhibiting higher inhibition level for all the biodeteriogenic fungi tested (Fig.2).

It was observed that the level of inhibition depended strongly on the biodeteriogenic fungal strain tested, with a particular potent effect against *P. glandicola* CCLBH-MP101.

### Example 7. Antifungal inhibitory capacity of Ly-Tech-1053 against fungus from marble slabs

With the purpose to evaluate real life efficiency and influence of the inventive compounds on the activity of biodeteriogenic microorganisms in marble samples, an assay using the bioactive Ly-Tech-1053 produced by *Bacillus sp.* CCLBH 1053 cultures in the stationary-phase of bacteria growth as described in Example 3 was performed under in situ controlled conditions.

After sterilization, marble slabs of 2 x 2 cm were inoculated with a mixture composed by 1 mL of *Penicillium glandicola* CCLBH-MP101 (MP), 105 CFU/mL spore suspension, respectively, 500 µL of malt extract and 500 µL of *Bacillus sp.* CCLBH 1053 liquid culture supernatant, containing the bioactive compounds produced, as described in the methodological scheme 1.

The slabs were incubated at 27°C, monitored periodically and documented using a digital camera. Control assays were also performed in the absence of the fungal suspension (MCP), in order to tested possible interference of the biocompounds of the invention in the structure of the marble slabs.

After 1 month of incubation all the slabs were air-dried, coated with gold and examined by Scanning Electron Microscopy (SEM) using a Hitachi S-3700N (Tokyo, Japan) variable pressure scanning electron microscope coupled to a Bruker XFlash 5010 energy dispersive X-ray (EDX) spectrometer (Berlin, Germany) to allow visualization of the surface "biofilm" and elemental composition (point analysis and two-dimensional mapping). The accelerating voltage was 18-20 kV.

### Example 8. In situ antifungal inhibitory capacity of Ly-Tech-1053 against fungus from marble slabs

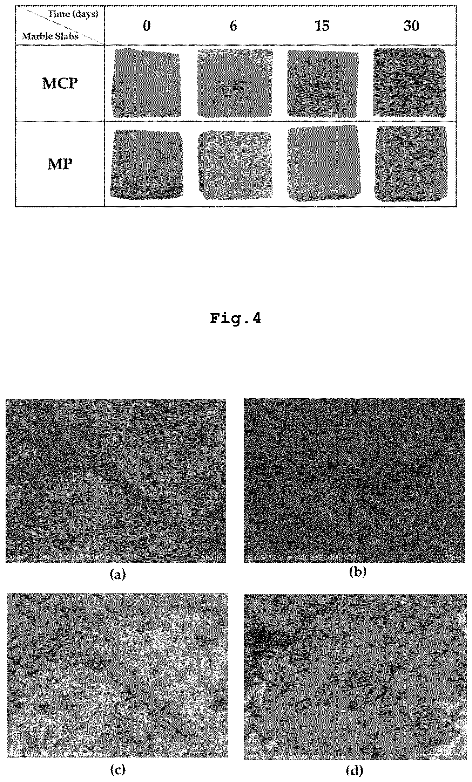

In order to develop and study the real efficiency and influence of the inventive compounds in the biodeteriogenic fungi development, an assay using marble slabs were envisaged. It was inoculated with *P. glandicola* CCLBH-MP101 and treated with Ly-Tech-1053 bioactive compounds, obtained as described in Example 3, under *in situ* controlled conditions. Figure 3 shows the macroscopic features corresponding to the marble slab assays, in the presence of *P. glandicola* CCLBH-MP101, a common fungi genera found in biodegraded artworks.

After 6 days of marble slab treatment, the positive control (MCP) begins to exhibit stains in the slab surface, marking the start of fungal proliferation (Fig.3).

At the end of the assay and after 30 days of incubation, the MCP slabs show evident contamination sports. On the contrary, the MP slabs, treated with the biocompounds of the invention, indicate visually a few signals of microbiological contamination in it surface and a marked difference with the slabs without antifungal treatment (MCP).

All marble slabs were analysed by Scanning Electron Microscopy (SEM) (Figure 4).

The MCP slabs (Figure 4a) exhibited a clear presence of microbial cells thriving in the stone. It was possible to observe spores and hyphae penetrating into the microstructure of the marble, promoting the proliferation of these microorganisms. Also, the EDX elemental analysis showed the presence of chemical elements such as carbon and oxygen, confirming the presence of organic material in the slabs (Figure 4c).

Relatively to the treated MP slabs with the biocompounds of the invention, a few evidences of fungal growth were observed by SEM (Figure 4b). The lack of fungal proliferation was confirmed by the EDX analyses that only showed the presence of marble nature composition elements, sodium (Na), chlorine (Cl) and calcium (Ca) (Figure 4d).

These simulation assays evidenced the great potential of the bacterial bioactive compounds to inhibit fungal proliferation in marble materials.

## Claims

1. A process for producing lipopeptides biocide compounds derived from *Bacillus sp.,* in particular from the strains selected from *Bacillus amyloliquefaciens* CCLBH 1051, CCLBH 1052 and CCLBH 1053, comprising the following steps:
a) Culturing and growing of *Bacillus amyloliquefaciens* strains CCLBH 1051, CCLBH 1052 or CCLBH 1053 in an adequate culture medium to induce nutritional stress, during 72h for producing LPP compounds;
b) Removal of Bacillus cells from the cultured medium;
c) Lyophilisation of the resulting cell-free supernatant of step (b);
d) Resuspension of the lyophilized product of (c) in a chloroform/methanol/water solvent-system solution to obtain a crude bioactive solution (Crude-C solution);
e) Chromatographic separation of lipopeptides bioactive compounds of the crude-C solution of step (d).

2. A process, according to claim 1 further comprising step (f) wherein the bioactive crude-C solution of step (d) is dried and subsequently lyophilized to obtain **bioactive compositions Ly-Tech-1051, Ly-Tech-1052 or Ly-Tech-1053,** respectively obtained from cultures of Bacillus sp. CCLBH 1051, CCLBH 1052 or CCLBH 1053.

3. A process, according to claim 2 further comprising step (g) wherein a bioactive composition of step (f) is added to polypropylene glycol 2000 and water, in a proportion of 400mg Ly-Tech composition to 2 ml polypropylene glycol 2000 (2%, v/v) and 998 water, to form BEVOTECH-11, BEVOTECH-14 and BEVOTECH-16 bioactive formulations, respectively comprising **Ly-Tech-1051, Ly-Tech-1052 and Ly-Tech-1053** as described in claim 2.

4. A process according to any of the claims 1 to 3 wherein the culture medium for inducing nutritional stress of step (a) has the following composition per litre: (NH4)2SO4, 1.5 g, KH2PO4, 1.7 g, Na2HPO4·2H2O, 1.7 g, MgSO4·7H2O, 0.2 g, yeast extract, 0.1 g, glucose 2 g and 2mL of micronutrients solution (MS), and wherein the MS composition is the following per litre: EDTA 25,0g, ZnSO4.7H2O 4,5g, MnCl2.4H2O 13,0g, CoCl2.6H2O 0,3g, CuSO4.5H2O 0,3g, NaMoO4.2H2O 0,4g, CaCl2.2H2O 4,5g, FeSO4.7H2O 3,0g, H3BO3 1,0g, and KI 0,1g.

5. A process according to any of the claims 1 to 3 wherein polypropylene glycol 2000 (2%, v/v) is added to the culture medium of step (a) for controlling the formation of foam.

6. A process according to claim 1 wherein the separation of lipopeptides in step (e) is performed by chromatography wherein the stationary phase is performed with flash silica gel 60 (0.04-0.06mm) in a glass column with 5cmx30cm, and the elution is performed at +/- 20ml/min, during +/- 10 minutes.

7. A bioactive crude-C solution obtainable by the process as described in any of the claims 1 to 6, comprising lipopeptides bioactive compounds.

8. A bioactive composition obtainable by the process as described in any of the claims 1 to 6, comprising lipopeptides bioactive compounds.

9. A bioactive formulation obtainable by the process as described in any of the claims 1 to 6, comprising lipopeptides bioactive compounds.

10. The use of a bioactive crude-C solution of claim 7, the bioactive composition of claim 8 or a bioactive formulation of claim 9 for controlling of biodegradation, biodeterioration and conservation of objects of cultural heritage.
